# EUROPEAN PATENT APPLICATION

(11) **EP 4 660 200 A1**
(43) Date of publication of application: **10.12.2025**
(21) Application number: 24179731.5
(22) Date of filing: 03.06.2024
(51) Int. Cl.: C07K 14/31, C12N 15/70

(54) **STABILIZED POLYNUCLEOTIDE**

(71) Applicant: MU Bioteknik Aktiebolag, 181 90 Lidingö (SE)
(72) Inventor: DAHLSSON LEITAO, Charles, 117 69 Stockholm (SE); UHLÉN, Mathias, 181 90 Lidingö (SE); STÅHL, Stefan, 111 40 Stockholm (SE); LÖFBLOM, John, 146 54 Tullinge (SE); NYGREN, Per-Åke, 178 40 Ekerö (SE); HOBER, Sophia, 115 46 Stockholm (SE)
(74) Representative: Kransell & Wennborg KB

(57) **Abstract**

The present disclosure provides a polynucleotide encoding a protein having n repeats of a Protein A-derived domain, wherein the polynucleotide comprises n domain-encoding regions, each domain-encoding region differs from the other domain-encoding regions by at least five synonymous codons and n is at least three.

## Description

### TECHNICAL FIELD

The present disclosure relates generally to the recombinant production of multimeric affinity ligands for bioseparation and specifically to the genetic stability of the polynucleotides encoding affinity ligands comprising repeats of a Protein A-derived domain for antibody purification.

### BACKGROUND

Affinity ligands in the form of multimers of a Protein A-derived domain are used in antibody purification columns. In these columns, the ligands are coupled to a matrix, such as Sepharose, which is a crosslinked, beaded form of agarose.

Protein A comprises five homologous Ig-binding domains called domain E, domain D, domain A, domain B and domain C. Domain B has attracted particular attention. Domain B has 58 amino acid residues. In mutated version of domain B referred to as domain Z, the A residue in position 1 is replaced with V and the G residue in position 29 is replaced with an A residue. The A1V mutation introduces an AccI restriction site. The G29A mutation is hydroxylamine-stabilizing (Asn-Gly, but not Asn-Ala, is hydroxylamine-sensitive). The G29A mutation also reduces the Fab-binding capability of the native B domain.

WO 03/080655 discloses versions of domain Z that have been mutated to improve the stability at elevated pH values. Such mutated versions have improved tolerance to typical cleaning conditions. According to WO 03/080655, N23T, N3A and N6D are particularly advantageous mutations when considering both affinity and alkali stability. Other asparagine-replacing mutations are also discussed.

WO 03/080655 also discusses multimers, such as dimers, trimers, tetramers and pentamers, of the mutated domain.

### SUMMARY

The present inventors have found that if identical polynucleotide sequences are used for each repeat of a Protein A-derived domain in a multimer affinity ligand, there is a problem with genetic instability in the form of transposon insertions and gene fragmentation.

The present inventors have also found that this problem can be reduced or even eliminated by silent mutations that introduce differences between the polynucleotide sequences that encode the respective domains. In other words, synonymous codons are used to create differences at the polynucleotide level that do not translate to structural differences between the domains at the protein level.

Accordingly, the present disclosure provides a polynucleotide encoding a protein having n repeats of a Protein A-derived domain, wherein the polynucleotide comprises n domain-encoding regions, each domain-encoding region differs from the other domain-encoding regions by at least five synonymous codons and n is at least three.

### BRIEF DESCRIPTION OF THE DRAWINGS

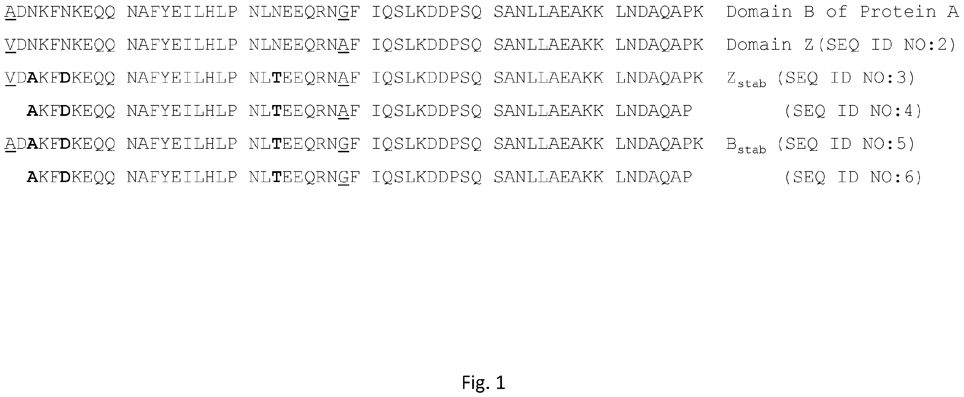

Figure 1 shows an alignment of:
   domain B of Protein A (SEQ ID NO:1);
   domain Z, which differs from domain B by having V instead of A in position 1 and A instead of G in position 29 (SEQ ID NO:2);
   Z_{stab}/SEQ ID NO:3, which is an alkali-stabilized version of domain Z;
   SEQ ID NO:4, which is a subsequence of Z_{stab};
   B_{stab}/SEQ ID NO:5, which is an alkali-stabilized version of domain B; and
   SEQ ID NO:6, which is a subsequence of B_{stab}.
   The underlined residues in Figure 1 highlight the differences between domain B and domain Z. The bold residues are mutations for improved stability under alkaline conditions.

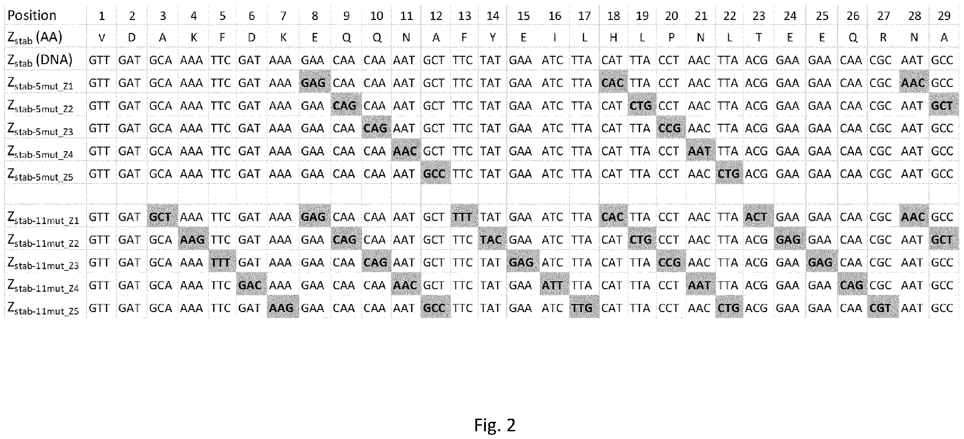

Figures 2-3 show the amino acid and the DNA sequence of Z_{stab} and the silent mutations with respect to the original Z_{stab} DNA sequence of the domain-encoding regions of two different inventive embodiments ("Z_{stab-5 mut}" and "Z_{stab-11 mut}"). In Z_{stab-11 mut} the differences between the domain-encoding regions have been maximized for a pentamer by introducing 11 unique silent mutations in each domain-encoding region such that each domain-encoding region differs from the other domain-encoding regions by 22 synonymous codons.

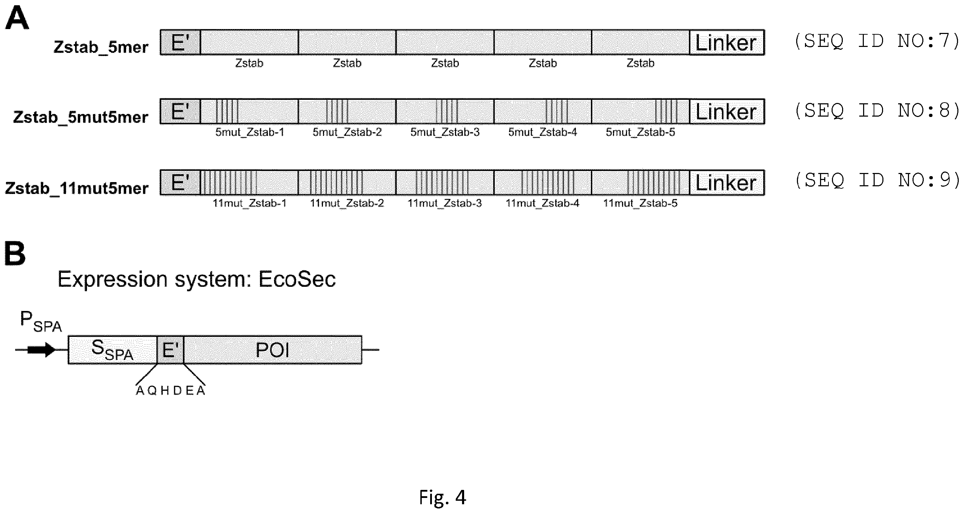

Figure 4A schematically illustrates three gene variants. Zstab_5mer comprises five genetically identical alkali stabilized Z domains, the E' sequence (AQHDEA, SEQ ID NO:10) upstream of the first Z domain and a linker sequence (GGGGSGGGGSC, SEQ ID NO:11) downstream of the fifth Z-domain followed by two stop codons. Zstab_5mut5mer and Zstab_nmutsmer encode the same amino acid sequence as Zstab_5mer, but have different genetic sequences. In the genes Zstab_5mut5mer and Zstab_nmutsmer, each Zstab domain has 5 and 11 unique silent mutations (illustrated by lines), respectively, compared to the consensus sequence of Zstab_5mer.
Figure 4B schematically illustrates the EcoSec expression system used for protein production. P_{SPA} is the promoter from the Protein A operon. The protein of interest (POI) in this case comprises the five Z_{stab} domains with the C terminal linker sequence. The Protein A operon-derived signal peptide sequence S_{SPA} direct the POI for secretion, allowing it to be recovered from the medium.

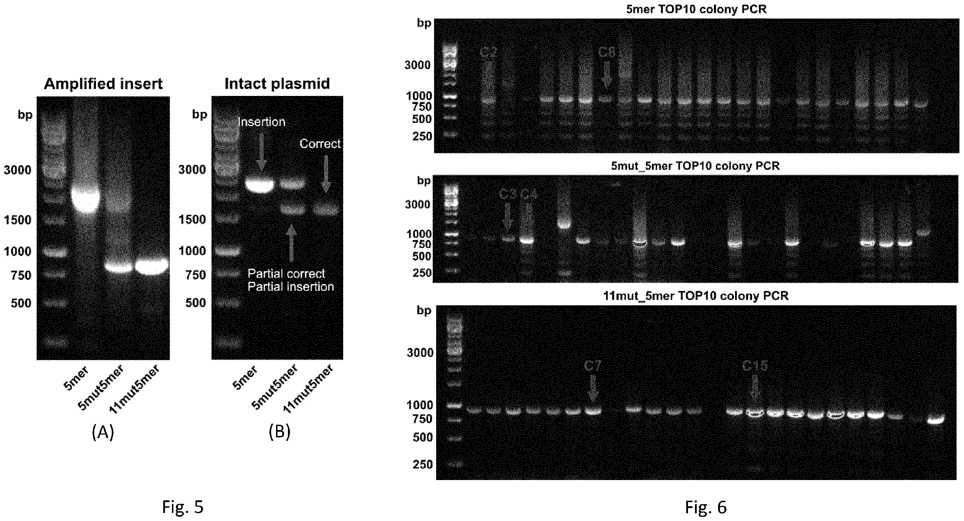

Figure 5 shows data from sequenced plasmids extracted from TOP10 cells. The left gel (A) shows the PCR-amplified insert and the right gel (B) shows the intact plasmid.
Figure 6 shows PCR screening of TOP10 colonies that were obtained after transformation using sequence-verified plasmids. 5mer and 5mut5mer exhibit colonies with transposon insertions and with multiple bands corresponding to the approximate sizes of inserts comprised of varying number of Zstab domains. The annotated arrows indicate which colonies were chosen for further investigation.

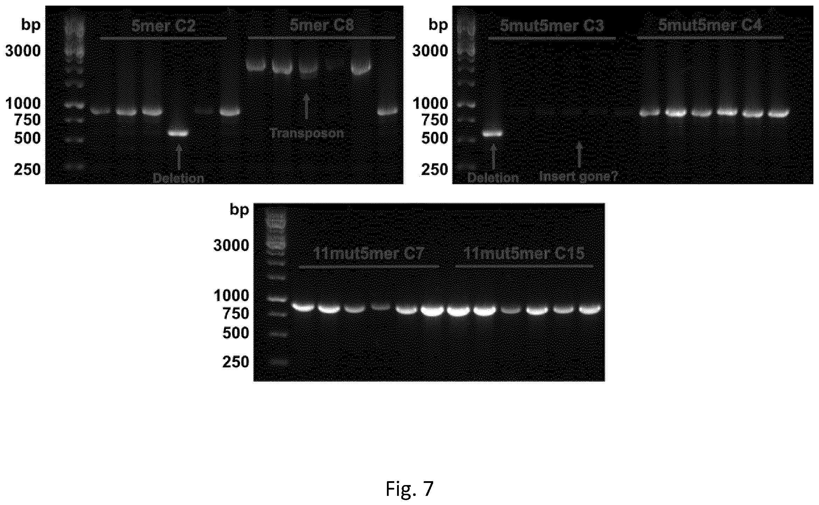

Figure 7 shows BL21(DE3) colony PCR screening following transformation using plasmids from colonies chosen during the TOP10 colony PCR screening. Deletions can be observed for both 5mer and 5mut5mer. Transposon insertions could be observed for all but one colony from the 5mer C8 track. No transposon insertions or deletions could be observed for nmutsmer.

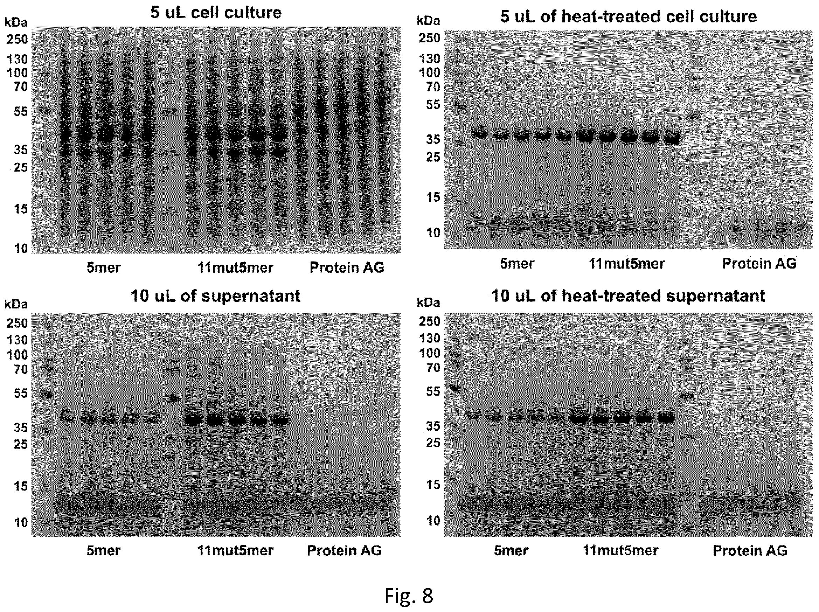

Figure 8 shows a comparison of protein production between 5mer, 11mut5mer and protein AG, all in the same EcoSec expression plasmid. The theoretical molecular weight for 5mer and 11mut5mer is approximately 38 kDa and for Protein AG it is approximately 54 kDa. Cell culture (both cells and medium) before and after heat-treatment are shown in the top panel, each well loaded with 5 µL sample. The supernatant (medium) before and after heat-treatment are shown in the bottom panel, each well was loaded with 10 µL sample. Five culture flask replicates of each variant were made.

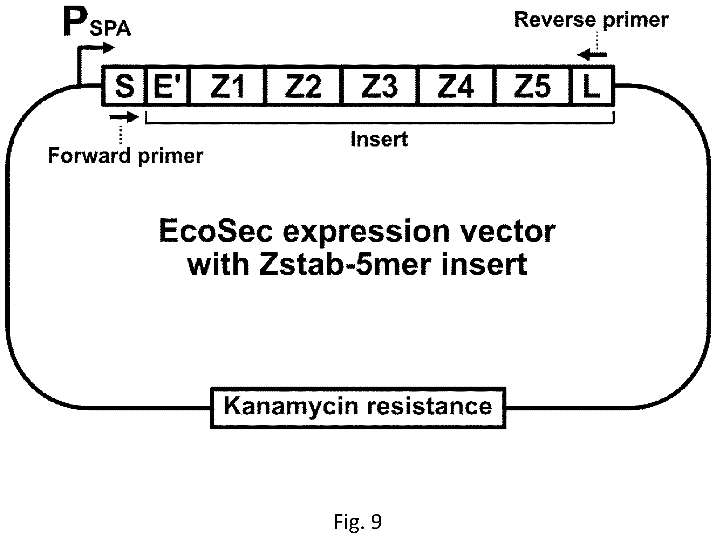

Figure 9 is a schematic illustration of the EcoSec expression plasmid containing the insert gene for Zstab-5mer. The positions of the forward and reverse primers used to amplify the insert during screening are shown. The insert comprises five Z-domains, E' and L. The Z-domains are flanked by the E'-sequence directly upstream of the first Z-domain, encoding the amino acids AQHDEA (SEQ ID NO:10), and a linker sequence (L) directly downstream of the last Z-domain, encoding the amino acids GGGGSGGGGSC (SEQ ID NO:11) followed by two stop codons. The signal peptide sequence (S) directs the protein for secretion.

### DETAILED DESCRIPTION

As a first aspect of the present disclosure, there is provided a polynucleotide encoding a protein having n repeats of a Protein A-derived domain, wherein the polynucleotide comprises n domain-encoding regions. As understood by the skilled person, the n domain-encoding regions encodes the n repeats of the Protein A-derived domain.

In the polynucleotide of the first aspect, each domain-encoding region differs from the other domain-encoding regions by at least five synonymous codons. For the avoidance of doubt, "synonymous codons" are defined as different codons that encode the same amino acid. Hence, the differences between the domain-encoding regions may be achieved by a pattern of silent mutations.

In the first aspect, n is at least three, such as three, four, five or six. Accordingly, the protein is a multimer, such as a trimer, tetramer, pentamer or hexamer. Preferably, n is five or six since such a relatively high number results in higher affinity density than three and four.

Preferably, each domain-encoding region differs from the other domain-encoding regions by at least ten synonymous codons. More preferably, each domain-encoding region differs from the other domain-encoding regions by at least 12, 14, 16, 18 or 20 synonymous codons.

In one embodiment, n is 5 and each domain-encoding region differs from the other domain-encoding regions by 22 synonymous codons. An example of such an embodiment is shown in figs. 2-3 (see Z_{stab-11 mut}).

In another embodiment, n is 6 and each domain-encoding region differs from the other domain-encoding regions by 18 synonymous codons.

A benefit of n being 5 instead of 6 is that more differences between each pair of domain encoding regions (i.e. unique silent mutations) can be created by synonymous codons.

The Protein A-derived domain is typically immunoglobulin-binding, such as IgG- -binding. An IgG-binding Protein A-derived domain is preferred.

Immunoglobulin-binding Protein A-derived domains typically binds the heavy chain within the Fc region of the immunoglobulin.

The Protein A-derived domain is preferably an optionally mutated B domain of Protein A. The B domain of Protein A has the following amino acid sequence: 

 (SEQ ID NO:1).
 Common mutations are A1V and G29A, which result in the following sequence:


 ("Z", SEQ ID NO:2).

In an embodiment, the Protein A-derived domain is a version of domain A or domain Z that has been mutated to improve stability at alkaline conditions. Examples of such mutations to SEQ ID NO:1 or 2 include N23T and optionally N3A and/or N6D. Preferably, domain B or domain Z has been mutated by each of N23T, N3A and N6D to form SEQ ID NO:5 or SEQ ID NO:3 (see Figure 1).

In one embodiment, the Protein A-derived domain is a so called affibody affinity protein. An affibody is a protein A-derived affinity protein developed using combinatorial protein engineering. In the literature, there are many examples of affibodies that have been engineered to bind different targets.

As an example, the Protein A-derived domain may comprise or consist of an amino acid sequence selected from: (i) SEQ ID NO:3; and (ii) a sequence which has at least 94% identity to SEQ ID NO:3.

As another example, the Protein A-derived domain may comprise an amino acid sequence selected from: (i) SEQ ID NO:4; and (ii) a sequence which has at least 94% identity to SEQ ID NO:4.

As another example, the Protein A-derived domain may comprise or consist of an amino acid sequence selected from: (i) SEQ ID NO:5; and (ii) a sequence which has at least 94% identity to SEQ ID NO:5.

As another example, the Protein A-derived domain may comprise an amino acid sequence selected from: (i) SEQ ID NO:6; and (ii) a sequence which has at least 94% identity to SEQ ID NO:6.

The term "% identity", as used throughout the present disclosure, is calculated as follows. The query sequence is aligned to the target sequence. A comparison is made over the window corresponding to the target sequence. The amino acid residues at each position are compared and the percentage of positions that exhibit identical amino acid residues in the query sequence and the target sequence is reported as % identity. As an example, SEQ ID NO:3 (query sequence) is 94.8% identical to SEQ ID NO:2 (target sequence) since there are different amino acid residues in 3 out of 58 positions. As another example, SEQ ID NO:4 (query sequence) is 94.8% identical to SEQ ID NO:3 (target sequence) since SEQ ID NO:4 is three amino acid residues shorter than SEQ ID NO:3, but otherwise identical to SEQ ID NO:3.

In an embodiment, the protein further comprises a linking sequence. The linking sequence is preferably provided at a terminal end of the protein, such as at the C terminal end of the protein. The length of the linking sequence may for example be 5-20 amino acid residues. An example of a suitable linking sequence is SEQ ID NO:11.

To facilitate coupling to a matrix, the linking sequence may comprise a cysteine (C) residue. The C residue is preferably the terminal residue of the linking sequence. Further, the C residue in of the linking sequence is preferably the only C residue of the protein.

In one embodiment, a terminal end (preferably the N terminal end) of the protein comprises an extension sequence. The extension sequence may consist of 3-15 amino acids, which preferably correspond to a N terminal sequence of the E domain of protein A. Such an extension sequence is associated with beneficial protein production properties. An example of a suitable extension sequence is SEQ ID NO:10.

In one embodiment, the polynucleotide of the first aspect comprises or consists of nucleotides 7-888 of SEQ ID NO:9.

In one embodiment, the polynucleotide of the first aspect comprises or consists of SEQ ID NO:9.

As a second aspect of the present disclosure, there is provided an expression vector, such as a plasmid, comprising a polynucleotide according to any one of the preceding claims.

As a third aspect of the present disclosure, there is provided a cell transfected or transformed with an expression vector according to the second aspect. The transfected cell is preferably a eucaryotic cell, such as a mammalian cell. The transformed cell is preferably a prokaryotic cell, such as an Escherichia coli cell.

As a fourth aspect of the present disclosure, there is provided an expression system comprising a polynucleotide according to the first aspect.

As a fifth aspect, there is provided a clone of cells comprising the polynucleotide of the first aspect.

### EXAMPLE

### Introduction

This example aims to generate a stable genetic construct coding for a purification ligand having five identical alkali-stabilized Z domains arranged consecutively. As discussed in the background section, the Z domain is derived from the B domain of staphylococcal protein A (SpA) by the introduction of two mutations (A1V and G29A) [1]. The Z domain binds to the Fc-region of immunoglobulin G, making it an extremely valuable and widely used tool for antibody purification in both industry and research [2]. Alkali-stabilization of the Z domain can be achieved by substituting three asparagine residues (N3A, N6D and N23t) in the sequence resulting in Z_{stab} (Figure 1, SEQ ID NO:3) [3].

Two genetic constructs, Z_{stab-5 mut 5mer} and Z_{stab-11 mut 5mer}, with 5 and 11 unique silent mutations, respectively, in each Z_{stab} domain have been compared to the genetic construct Z_{stab 5mer} composed of genetically identical domains (see Figure 4A). Henceforth, the three variants will be referred to as 5mer, 5mut5mer and 11mut5mer for simplicity. In 5mut5mer, each domain-encoding region differs from the other domain-encoding regions by ten synonymous codons (see Figures 2-3). In 11mut5mer, each domain-encoding region differs from the other domain-encoding regions by 22 synonymous codons (see Figures 2-3). The silent mutations were introduced to reduce the similarity between domains and to prevent genetic instability associated with repetitive gene sequences, such as promoting recombination events leading to deletions or insertions. The genetic variants have been evaluated in a plasmid which uses the EcoSec expression system consisting of a constitutively active promoter (P_{SPA}) and a signal peptide sequence (S_{SPA}) from *Staphylococcus aureus* (Figure 4B) [4]. The signal peptide sequence targets the protein to the general secretory pathway, allowing it to be recovered from the periplasm and/or culture medium. Additionally, the E' sequence is derived from the N terminal of the E domain of SpA, which in the bacterium appears immediately downstream of P_{SPA}, with benefits to protein production.

### Materials and methods

### Cloning into the EcoSec expression plasmid

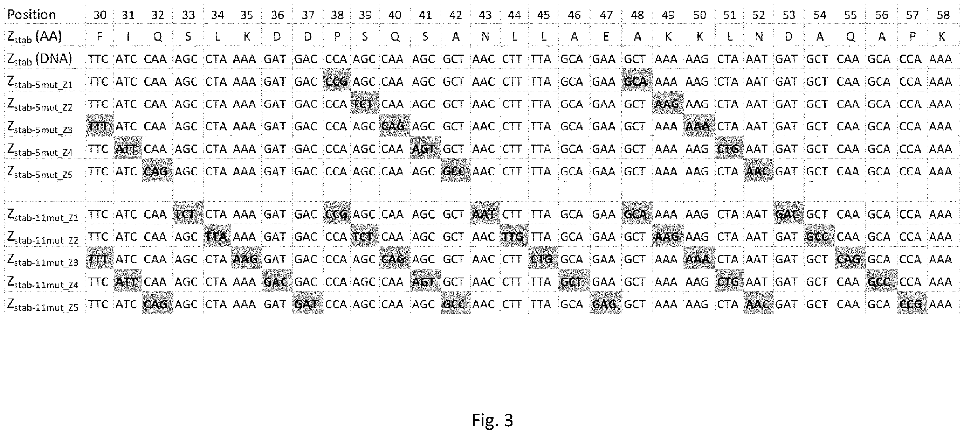

The DNA sequence variants corresponding to the 5mer (SEQ ID NO:7), 5mut5mer (SEQ ID NO:8) and 11mut5mer (SEQ ID NO:9) sequences, schematically depicted in Figure 4A, were synthesised (GenScript) and arrived as inserts in PUC57 plasmids. The exact domain-coding sequences are shown in Figures 2 and 3. Each insert was amplified by a standard PCR protocol using Phusion High-Fidelity DNA polymerase (New England Biolabs, Ipswich, MA, USA) and primers annealing to the ends of the inserts.

Linearisation of the EcoSec expression plasmid was performed by PCR amplification using Phusion High-Fidelity DNA polymerase (New England Biolabs) and primers with overhangs specific for the ends of the synthesised inserts. The PCR product was pooled, mixed with CutSmart buffer (New England Biolabs) and treated with DpnI (New England Biolabs) at 37 °C for 30 min, followed by heat-inactivation at 80 °C for 20 min.

The amplified inserts for 5mer, 5mut5mer and 11mut5mer were cloned into the linearised EcoSec expression plasmid using InFusion cloning (Takara Bio, Kusatsu, Japan) according to the recommended protocol (Figure 9). The InFusion reaction mixture was used to transform TOP10 competent cells (Thermo Fisher Scientific, Waltham, MA, USA) using a standard heat-shock protocol. Following recovery in SOC medium at 37 °C for 1 hour, the transformed cells were spread on agar plates containing kanamycin and incubated at 37 °C overnight. A single colony for each variant was used to inoculate 10 mL TSB-Y (Tryptic soy broth and yeast extract, Merck) supplemented with 50 ng/mL kanamycin. The following day, plasmids were extracted using a Qiagen Plasmid Mini Kit (Qiagen, Hilden, Germany) and sent for Full Plasmid Sequencing (Microsynth Seqlab, Gottingen, Germany).

### Evaluation of genetic stability

The intact plasmids and the inserts, amplified by PCR, were analysed on a 1% agarose gel running at 100 V for 60 min. The plasmids were used to transform BL21(DE3) competent cells (Thermo Fisher Scientific) with a standard heat-shock protocol. The visual distinction of colonies harbouring plasmids with transposons allowed for the isolation and extraction of plasmids with the correct insert lacking the transposon, which was also confirmed by Sanger sequencing (Microsynth Seqlab, Gottingen, Germany).

New TOP10 cells were transformed with plasmids containing the correct inserts using standard heat-shock protocol. For each variant, 25 colonies were screened using colony PCR. Briefly, colonies were picked using pipette tips and added to wells containing 10 µL water in a 96-well plate, which was subsequently heated to 90 °C for 10 min. Insert amplification from each colony was performed using a standard PCR protocol in a reaction volume of 20 µL with Taq DNA polymerase (New England Biolabs) and 1 µL of the heated colony samples.

Based on the colony PCR screening, two colonies for each variant were picked to inoculate 10 mL TSB-Y supplemented with 50 ng/mL kanamycin and incubated overnight at 37 °C. Plasmids were extracted from the overnight cultures using Qiagen Plasmid Mini Kit (Qiagen) and used to transform Bl21(DE3) cells using standard heat-shock protocol. Five colonies from each variant were screened using colony PCR.

### Evaluation of protein expression

Protein production using the EcoSec expression plasmid was evaluated for 5mer, 11mut5mer and protein AG in BL21(DE3) cells. For each variant, 10 mL of TSB-Y culture medium supplemented with 50 ng/mL kanamycin was inoculated from glycerol stocks of cells harbouring plasmids with sequences verified to be correct by Full Plasmid Sequencing (MicroSynth Seqlab), and incubated overnight at 37 °C. The following day, four samples were prepared from each overnight culture and analysed using SDS-PAGE (NuPage, Invitrogen). These samples comprised cell culture (cells and medium), heat-treated cell culture, supernatant (following centrifugation and removal of cells), and heat-treated supernatant. Heat-treated samples were heated to 95 °C for 10 min, put on ice for 20 min followed by removal of protein aggregates by centrifugation.

### Results

### Evaluation of genetic stability

The sequencing results revealed the presence of inserted DNA corresponding to an IS4-like element ISVsa5 family transposase. This was also confirmed by transforming new TOP10 cells and evaluating the size of the insert and intact plasmid on an agarose gel (Figure 5), showing that the insertion was present in nearly the entire plasmid population of 5mer, but only in half the plasmid population of 5mut5mer. No insertions were detected in 11mut5mer. BL21(DE3) cells were transformed with the sequenced plasmids (containing the transposon insertion, in the case of 5mer and 5mut5mer). The resulting colonies for 5mer and 5mut5mer harbouring the transposon insertion exhibited two distinct sizes corresponding to plasmids with transposon (large colony size) and without transposon (small colony size).

The plasmids verified to be correct by sequencing were transformed into TOP10 cells and colonies were screened by PCR (Figure 6). From the screening, some of the colonies for 5mer and 5mut5mer exhibited additional bands of smaller size, which could be an indication of recombination-mediated deletion. Two colonies (one exhibiting additional bands and one which did not) from each of 5mer (C2 and C8), 5mut5mer (C3 and C4) and 11mut5mer (C7 and C15) were picked and grown overnight at 37°C. The plasmids were extracted, sent for sequencing, and retransformed into BL21(DE3) cells (Figure 7). It was hypothesised that if additional bands correspond to plasmids with recombinant-mediated deletions of the inserts, then by retransforming the plasmids we should be able to isolate and observe these deletions. For one of the colonies from 5mer C2 and 5mut5mer C3 deletion was observed, which suggests that recombination occurred. Another instance of transposon insertion was observed for 5mer C8. No deletions or transposon insertions were observed for nmutsmer.

### Evaluation of protein expression

Protein production was compared from overnight cultures of BL21(DE3) cells at 37°C using the EcoSec expression system. Comparisons between 5mer, 11mut5mer and protein AG (a previously evaluated fusion between protein A and protein G [5] were made (Figure 8). Cell culture (cells and medium) and supernatant (medium), with and without the use of heat-treatment, were evaluated on SDS-PAGE. Heat-treatment did not seem to affect the amount of protein present in the sample, meaning the Zstab_5mer protein is capable of refolding following heat-denaturation. An interesting observation is that higher amounts of total protein is produced and secreted into the medium by the 11mut5mer compared to the 5mer clone, showing that repetitive sequences affect protein expression levels and secretion.

### References

[1] Henrichson, T.A. Jones, M. Uhlén, A synthetic IgG-binding domain based on staphylococcal protein a, Protein Eng. Des. Sel. 1 (1987) 107-113. https://doi.org/10.1093/protein/1.2.107.
[2] S. Kanje, J. Scheffel, J. Nilvebrant, S. Hober, Engineering of Protein A for improved purification of antibodies and Fc-fused proteins, Approaches to Purification, Anal. Charact. Antibody-Based Ther. (2020) 35-54. https://doi.org/10.1016/B978-0-08-103019-6.00002-3.
[3] J. Feldwisch, V. Tolmachev, C. Lendel, N. Herne, A. Sjöberg, B. Larsson, D. Rosik, E. Lindqvist, G. Fant, I. Höidén-Guthenberg, J. Galli, P. Jonasson, L. Abrahmsén, Design of an Optimized Scaffold for Affibody Molecules, J. Mol. Biol. 398 (2010) 232-247. https://doi.org/10.1016/j.jmb.2010.03.002.
[4] S. Josephson, R. Bishop, Secretion of peptides from E. coli: a production system for the pharmaceutical industry, Trends Biotechnol. 6 (1988) 218-224. https://doi.org/10.1016/0167-7799(88)90077-7.
[5] M. Eliasson, A. Olsson, E. Palmcrantz, K. Wiberg, M. Inganas, B. Guss, M. Lindberg, M. Uhlen, Chimeric IgG-binding receptors engineered from staphylococcal protein A and streptococcal protein G, J. Biol. Chem. 263 (1988) 4323-4327. https://doi.org/10.1016/50021-9258(18)68928-8.

## Claims

1. A polynucleotide encoding a protein having n repeats of a Protein A-derived domain, wherein the polynucleotide comprises n domain-encoding regions, each domain-encoding region differs from the other domain-encoding regions by at least five synonymous codons and n is at least three.

2. The polynucleotide of claim 1, wherein each domain-encoding region differs from the other domain-encoding regions by at least ten synonymous codons.

3. The polynucleotide of claim 2, wherein each domain-encoding region differs from the other domain-encoding regions by at least 12, 14, 16, 18 or 20 synonymous codons.

4. The polynucleotide of claim 3, wherein n is 5 and each domain-encoding region differs from the other domain-encoding regions by 22 synonymous codons.

5. The polynucleotide of claim 3, wherein n is 6 and each domain-encoding region differs from the other domain-encoding regions by 18 synonymous codons.

6. The polynucleotide of any one of the preceding claims, wherein the Protein A-derived domain is immunoglobulin-binding.

7. The polynucleotide of claim 6, wherein the Protein A-derived domain binds an Fc fragment of an immunoglobulin, such as IgG, IgA and/or IgM.

8. The polynucleotide of any one of the preceding claims, wherein the Protein A-derived domain is an optionally mutated B domain of Protein A.

9. The polynucleotide of any one of the preceding claims, wherein the Protein A-derived domain is alkali-stabilized.

10. The polynucleotide of any one of the preceding claims, wherein the Protein A-derived domain comprises an amino acid sequence selected from: (i) SEQ ID NO:4; and (ii) a sequence which has at least 94% identity to SEQ ID NO:4.

11. The polynucleotide of any one of the preceding claims, wherein the Protein A-derived domain comprises an amino acid sequence selected from: (i) SEQ ID NO:3; and (ii) a sequence which has at least 94% identity to SEQ ID NO:3.

12. The polynucleotide of any one of the preceding claims, wherein the protein further comprises a linking sequence at a terminal end of the protein, which linking sequence preferably comprises a cysteine residue.

13. An expression vector, such as a plasmid, comprising a polynucleotide according to any one of the preceding claims.

14. A cell transfected or transformed with an expression vector according to claim 13.

15. An expression system comprising a polynucleotide according to any one of claims 1-12.
